# EUROPEAN PATENT APPLICATION

(11) **EP 1 790 375 A1**
(43) Date of publication of application: **30.05.2007**
(21) Application number: 05770820.8
(22) Date of filing: 11.08.2005
(51) Int. Cl.: A61M 37/00, A61N 1/30

(54) **TRANSDERMAL DRUG ADMINISTRATION APPARATUS WITH MICRONEEDLE**

(30) Priority: 12.08.2004 JP 2004235629
(71) Applicant: HISAMITSU PHARMACEUTICAL CO. INC., Tosu-shi, Saga-ken 841-0017 (JP)
(72) Inventor: ADACHI, Hirotoshi Tsukuba Lab.Hisamitsu Pharm.Co.Inc., Tsukuba-shi, Ibaraki 3050856 (JP); TOKUMOTO, Seiji Tsukuba Lab.Hisamitsu Phm.Co.Inc., Tsukuba-shi, Ibaraki 3050856 (JP); HIGO, Naruhito Tsukuba Lab.HisamitsuPharm.Co.Inc., Tsukuba-shi, Ibaraki 3050856 (JP)
(74) Representative: Westendorp, Michael Oliver
(86) International application number: PCT/JP2005/014738
(87) International publication number: WO 2006/016647

(57) **Abstract**

A transdermal drug administration system with microneedles which can perforate the skin (stratum corneum) by a simple operation at the time of transdermal administration of a physiologically active substance (drug) is provided.

The present transdermal drug administration system with microneedles comprises microneedle device (50) having a plurality of microneedles (51) which can perforate the skin and a plurality of solution passages (52); absorbent (11) which is disposed on microneedle device (50), comprising dried drug (10) and a material which can absorb liquid; and dissolution liquid reservoir (18) which is disposed on absorbent (11) and stores dissolution liquid (16) for dissolving drug (10) and in which a diaphragm (20) provided between absorbent (11) can be broken by pressing. Dissolution liquid reservoir (18) is pressed to break diaphragm (20), and at the same time, the microneedle (51) perforates the skin (stratum corneum), and drug (10) dissolved in dissolution liquid (16) is transmitted through microneedle device (50) to the skin.

## Description

### Technical Field

The present invention relates to a transdermal drug administration system for administering a drug through the skin, particularly to a transdermal drug administration system with microneedles which comprises a plurality of microneedles capable of perforating the skin.

### Background Art

Conventionally, a method for administering a drug by adhering a drug-containing patch to the skin and allowing the drug to infiltrate into the skin from this patch is commonly performed. In the meantime, as a method for promoting absorption of a drug through the skin and mucous membrane, administrating methods using electric energy such as iontophoresis (Journal of Pharmaceutical Sciences, Vol. 76, p. 341, 1987) and electroporation (National Publication of International Patent Application No. 1991-502416, Proc.Natl. Acad. Sci. USA, Vol. 90, pp. 10504-10508, 1993) have been developed. Both the iontophoresis and electroporation are expected to be used as a method for promoting transdermal or transmucosal absorption of a drug.

In relation to promotion of drug absorption, National Publication of International Patent Application No. 2000-512529 (Patent Document 1) proposes a device which mechanically perforates the skin before releasing a transdermal pharmaceutical agent and thereby enhances the transdermal flow. This device has a sheet having a plurality of openings ; a plurality of microblades which are incorporated therewith and extend downward therefrom; and means to anchor the device on the body surface. In this case, the drug form serving as a reservoir for the pharmaceutical agent is, for example, a viscous gel.

Among these types of devices, those capable of retaining a drug in dry form include, for example, a device having skin needles for transdermally administering a protein or a peptide drug described in Japanese Patent Publication No. 6-14980 (Patent Document 2). In this device, an electrode leading to the outside, a polymer electrolyte reservoir, a drug support of hydrophilic polymer and a skin needle support of water swellable polymer are laminated, and a solvent inlet is formed in the central part at the upper end of the polymer electrolyte reservoir. This solvent inlet is formed of rubber and the like, for example, in the form of V-ditch so that an ionized solvent composition can be poured into the polymer electrolyte reservoir with a syringe and the like. When this device is used, it is necessary to separately prepare a syringe or the like for injecting a solvent composition.

As a device which is capable of retaining a drug in dry form and does not require a syringe or the like, there is, for example, a transdermal delivery device with a valve described in WO03/084595A1 (Patent Document 3). This device has a reservoir capable of retaining, for example, distilled water; a valve opening and closing this reservoir; a cavity capable of retaining a dried drug; and a plurality of minute skin penetration members which can penetrate the skin. This device is placed on the skin of a patient in time of use, pressed downward so that the minute skin penetration members can penetrate the skin, and then opens the valve, presses the reservoir and supplies the distilled water to the dried drug, thereby delivering the drug to the patient.

On the other hand, a new plaster structural body for iontophoresis is disclosed in Japanese Patent Publication No. 5-84180 (Patent Document 4) but it does not have such a skin needle as mentioned above. This structural body is provided with a capsule encapsulating, for example, an electrolytic solution in the upper part of the plaster structural body, and has a structure so that a film such as aluminum foil disposed between this capsule and a water containing layer can be broken to impregnate the electrolytic solution when it is attached. And it is described that when a water-decomposable drug is used, it is preferable to keep the drug containing layer and the water containing layer adjusted in a dry state and to provide the drug as a plaster structural body having a capsule encapsulating an electrolytic solution.
Patent Document 1: National Publication of International Patent Application No. 2000-512529
Patent document 2: Japanese Patent Publication No. 6-14980
Patent document 3: WO03/084595A1
Patent document 4: Japanese Patent Publication No. 5-84180

### Disclosure of the invention

As stated above, when a drug in a dry state is held in a transdermal drug administration system having skin needles, it has been conventionally necessary to separately prepare a syringe or the like for supplying the liquid to the drug, or provide a valve for supplying a liquid in the device. It is cumbersome to separately prepare a syringe in the use of a device and there is a case where it is difficult for a patient to operate the syringe. In addition, providing a valve for liquid supply in a device complicates the device and increases the cost.

Therefore, an object of the present invention is to provide a transdermal drug administration system with microneedles which can perforate the skin (stratum corneum) in a simple operation at the time of transdermal administration of a physiologically active substance (drug).

The object can be achieved by a transdermal drug administration system with microneedles which comprises a microneedle device having a plurality of microneedles which can perforate the skin and a microneedle substrate having at least one solution passage; a pad part disposed on the microneedle device; and a dissolution liquid reservoir disposed on the pad part and storing dissolution liquid for dissolving a drug, wherein a dried drug is placed in the pad part or microneedle device, and the dissolution liquid reservoir is pressed to open the dissolution liquid reservoir from a sealed state to supply the dissolution liquid to the pad part and to allow the microneedles to perforate the stratum corneum of the skin, thereby enabling the drug dissolved in the dissolution liquid to be transdermally absorbed. Here, an electrode can be provided on the pad part in order to supply electric energy from an external part. In addition, a sonic oscillator can be provided on the pad part in order to supply sound vibration energy from an external part. The microneedle device can possess a plate-like reinforcing member having at least one solution passage on the microneedle substrate. In addition, the pad part can possess a drug retaining member which contains the dried drug and an absorbent which absorbs the dissolution liquid.

In addition, a transdermal drug administration system with microneedles of the present invention comprises a microneedle device comprising a plurality of microneedles which can perforate the skin and amicroneedle substrate having at least one solution passage; an absorbent which is placed on the microneedle device, can contain the above dried drug and comprises a material which can absorb the liquid; and a dissolution liquid reservoir which is disposed on the absorbent and stores dissolution liquid for dissolving the drug and in which a diaphragm provided between the reservoir and the absorbent can be broken by pressing.

In addition, a transdermal drug administration system with microneedles of the present invention comprises a microneedle device comprising a plurality of microneedles which can perforate the skin and a microneedle substrate having at least one solution passage; a drug retaining member which is disposed on the microneedle device and contains a dried drug; an absorbent which is placed on the drug retaining member and comprises a material which can absorb the liquid; and a dissolution liquid reservoir which is disposed on the absorbent and stores dissolution liquid for dissolving the drug and in which a diaphragm provided between the reservoir and the absorbent can be broken by pressing.

Here, an electrode can be provided on the absorbent in order to supply electric energy from an external part. This enables the device to be used as a device for electric drug administration system, for example, a device for iontophoresis system (iontophoresis electrode structural body). In addition, a sonic oscillator can be provided on the absorbent in order to supply sound vibration energy from an external part. In this case, a plurality of the microneedles have a hollow passage which can transmit the drug in the direction along the length, and the hollow passage of the microneedle can be connected with a solution passage of the microneedle substrate. Furthermore, the microneedle device can possess a skin fixation part on the outside to extend the skin.

In addition, a transdermal drug administration system with microneedles of the present invention comprises a microneedle device comprising a plurality of microneedles which can perforate the skin and amicroneedle substrate having at least one solution passage; a dissolution liquid reservoir which is disposed on the microneedle substrate and stores dissolution liquid for dissolving the drug, wherein a dried drug is placed in the microneedle device, and the dissolution liquid reservoir is pressed to open the dissolution liquid reservoir from a sealed state to supply the dissolution liquid to the microneedle device and to allow the microneedles to perforate the stratum corneum of the skin, thereby enabling the drug dissolved in the dissolution liquid to be transdermally absorbed. Here, the dissolution liquid can be supplied to the microneedle through at least one solution passage formed on the microneedle substrate. In addition, the dissolution liquid can be supplied to the microneedle from the circumference of the microneedle substrate. Furthermore, an absorbent composed of a material which can absorb liquid can be provided at least on the part where the dissolution liquid reservoir is opened between the microneedle device and the dissolution liquid reservoir.

The transdermal drug administrationmethod of the present invention comprises applying a device which comprises a microneedle device having a plurality of microneedles which can perforate the skin; a pad part disposed on the microneedle device; a dissolution liquid reservoir disposed on the pad part and storing dissolution liquid for dissolving a drug; and drug disposed in the pad part or the microneedle device to the skin, and pressing the dissolution liquid reservoir to open the dissolution liquid reservoir from a sealed state to supply the dissolution liquid to the pad part and to allow the microneedles to perforate the stratum corneum of the skin, thereby transdermally administering the drug dissolved in the dissolution liquid through the microneedles.

In addition, the transdermal drug administration method of the present invention comprises applying a device which comprises a microneedle device having a plurality of microneedles which can perforate the skin; a dissolution liquid reservoir disposed on the microneedle device and storing dissolution liquid for dissolving a drug; and drug disposed in the microneedle device to the skin, and pressing the dissolution liquid reservoir to open the dissolution liquid reservoir from a sealed state to supply the dissolution liquid to the microneedles and to allow the microneedles to perforate the stratum corneum of the skin, thereby transdermally administering the drug dissolved in the dissolution liquid through the microneedles.

In the present invention, this device is attached to the skin at first and a plurality of microneedles are contacted against the horny surface of the skin in time of use. And the dissolution liquid reservoir containing dissolution liquid is opened from a sealed state by pressing the dissolution liquid reservoir (container). This allows the dissolution liquid to flow into the microneedle device through a pad part or an absorbent or directly and dissolve a physiologically active substance (drug) in the dissolution liquid, and allow the microneedles to perforate the stratum corneum when pressing the dissolution liquid reservoir and the drug dissolved in the solution passes through the perforated openings and is transdermally absorbed by the skin. Energy is added to promote transdermal absorption of a drug if necessary afterwards.

According to the invention, a transdermal drug administration system with microneedles which can perforate the skin (stratum corneum) by a simple operation at the time of transdermal administration of a physiologically active substance (drug) can be provided. Treatment effect by transdermal administration (passive diffusion) or iontophoresis of a physiologically active substance can be enhanced by perforating the skin (stratum corneum) with microneedles at the time of transdermal administration of a physiologically active substance.

### BRIEF DESCRIPTION OF THE DRAWINGS

Figure 1 is a schematic view showing an example of a transdermal drug administration system with microneedles of the present invention.
Figure 2 is a drawing showing one example of a transdermal drug administration system with microneedles of the present invention. (a) is a plan view, (b) is a cross-section view along X-X and (c) and (d) are drawings of the device of the present invention at use.
Figure 3 is a drawing showing another example of a transdermal drug administration system with microneedles of the present invention. (a) is a plan view, (b) is a cross-section view along X-X and (c) and (d) are drawings of the device of the present invention at use.
Figure 4 is a drawing showing another example of a transdermal drug administration system with microneedles of the present invention.
Figure 5 is a drawing showing another example of a transdermal drug administration system with microneedles of the present invention.
Figure 6 is a drawing showing a construction example of a microneedle device to be used in the transdermal drug administration system with microneedles of the present invention, and (a) is a whole view, (b) is a enlarged view of a part surrounded with a dotted line in (a), and (c) is a partially enlarged view showing a modified example of a microneedle device.
Figure 7 is a drawing showing another example of a transdermal drug administration system with microneedles of the present invention.
Figure 8 is a drawing showing another example of a transdermal drug administration system with microneedles of the present invention.
Figure 9 is a drawing showing another example of a transdermal drug administration system with microneedles of the present invention.
Figure 10 is a drawing showing another example of a transdermal drug administration system with microneedles of the present invention.
Figure 11 is a drawing showing another example of a transdermal drug administration system with microneedles of the present invention.

### Description of symbols

- 10: Drug
- 11: Absorbent which contains dried drug
- 12: Adhesive layer
- 13: Wall member
- 14: Opening
- 15: Support
- 16: Dissolution liquid
- 17: Protruding part
- 18: Dissolution liquid reservoir
- 19: Liner
- 20: Diaphragm
- 25: Electrode
- 26, 61: Leads part
- 31: Absorbent which does not contain drug
- 32: Drug retaining member
- 41: Pad part
- 50: Microneedle device
- 51, 56: Microneedle
- 52: Solution passage
- 53: Microneedle substrate
- 54: Skin
- 55: Pressing direction
- 57: Hollow passage
- 58: Skin fixation part
- 59: Plate-like reinforcing member
- 60: Sonic oscillator

### Best Mode for Carrying Out the Invention

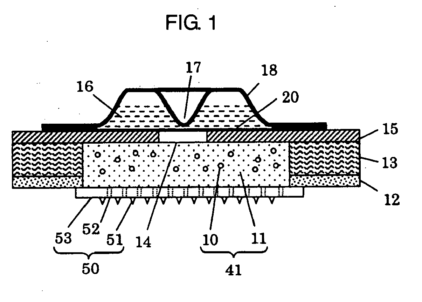

Figure 1 is a schematic view showing an example of a transdermal drug administration system with microneedles of the present invention. This device, as shown by the drawing, comprises microneedle device 50 having a plurality of microneedles 51 which can perforate the skin and microneedle substrate 53 having at least one solution passage 52; pad part 41 disposed on the microneedle device 50; and dissolution liquid reservoir 18 which is disposed on pad part 41, stores dissolution liquid 16 for dissolving a drug and can be opened by pressing. Pad part 41, as in this example, can possess absorbent 11 consisting of a material which can absorb liquid and drug 10. Disposition of drug 10 is not limited to this. It can be disposed in a drug retaining member or a microneedle device as described later. Wall member 13 having adhesive layer 12 on the bottom surface is disposed around absorbent 11, and support 15 having opening 14 is disposed on absorbent 11 and wall member 13, and diaphragm 20 is disposed on support 15. Diaphragm 20 may be formed separately from dissolution liquid reservoir 18 or may be formed as one body. Dissolution liquid reservoir 18 has protruding part 17 to facilitate destruction of diaphragm 20.

At the time of use, this device is attached to the skin to contact microneedle 51 against the stratum corneum of the skin. And diaphragm 20 is destroyed with protruding part 17 by pressing dissolution liquid reservoir 18. This opens dissolution liquid reservoir 18 from a sealed state and allows microneedles 51 to perforate the stratum corneum of the skin by the pressing and thereby transdermally administering the drug dissolved in dissolution liquid 16.

An electrode and a lead part can be provided on pad part 41 of this device, which enables the device to be used as a device for electric drug administration system, for example, a device for iontophoresis system (iontophoresis electrode structural body). When this device is used as a normal patch, the electrode is not necessary. In addition, in this device, pad part 41 can separately possess an absorbent consisting of a material capable of absorbing liquid and a drug retaining member containing a drug. In addition, drug can be disposed not in pad part 41 but in microneedle device 50. In this case, the drug can be disposed outside or within the hollow passage of microneedle 51 or on microneedle substrate 53 or solution passage 52.

Hereinbelow, examples of the present invention are described in detail.

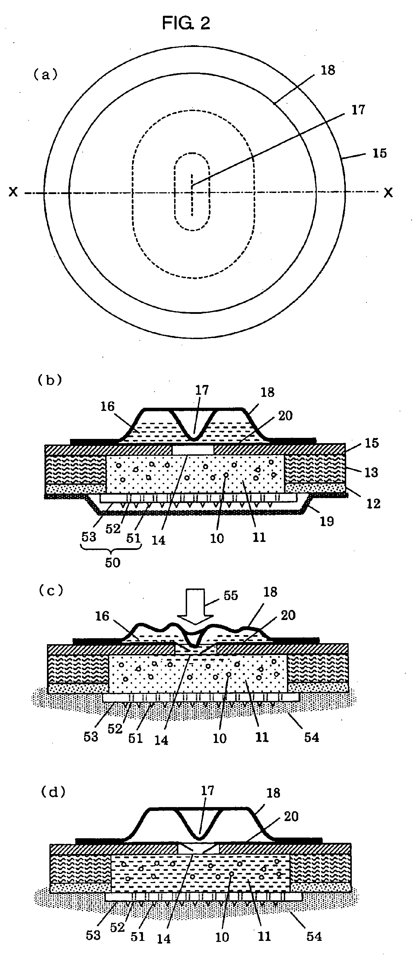

Figure 2 is a drawing showing an example of a transdermal drug administration system with microneedles of the present invention. (a) is a plan view, (b) is a cross-section view along X-X and (c) and (d) are drawings of the device of the present invention at use. The device of this example can be used, for example, as a normal patch and, as shown in Figs 2 (a) and (b), comprises microneedle device 50 having a plurality of microneedles 51 which can perforate the skin and microneedle substrate 53 having a plurality of solution passages 52; absorbent 11 which is disposed on microneedle device 50 and composed of a material capable of containing dried drug 10 and absorbing liquid; wall member 13 having adhesive layer 12 on the bottom surface arranged around absorbent 11; support 15 which has opening 14 in the center and is disposed on absorbent 11 and wall member 13; diaphragm 20 disposed on support 15; and dissolution liquid reservoir 18 disposed on diaphragm 20, retaining dissolution liquid 16 which dissolves a drug inbetween diaphragm 20 and having protruding part 17 for destroying diaphragm 20. Protruding part 17 has, for example, a linear tip as shown, and is disposed in contact with or in the vicinity of diaphragm 20. Liner 19 is removably attached on the bottom of microneedle device 50 and adhesive layer 12. Here, dissolution liquid reservoir 18 and diaphragm 20 may be formed separately or may be formed as one body. The shape of opening 14 of the support is not particularly limited, and it is enough that it is a shape which can thoroughly supply solution to absorbent 11, and preferably, for example, a round form. In this case, dimensions of opening 14 depend on the size of absorbent 11, but, for example, it has a diameter of 2 mm to 10 mm, and preferably 4 mm to 8 mm. Support 15 can be omitted by making diaphragm 20 to also perform the function thereof. In this case, no opening is provided, and opening will be formed in time of use beforehand by a protruding part. Diaphragm 20 can be also formed as a part of dissolution liquid reservoir 18.

Liner 19 is removed in time of use as shown in Figure 2 (c) and this device (patch) is adhered onto skin 54. And the top surface of dissolution liquid reservoir 18 is pressed in direction of arrow 55 to break diaphragm 20 with protruding part 17. At this time, diaphragm 20 is largely broken along the linear tip of protruding part 17 and dissolution liquid 16 in dissolution liquid reservoir 18 flows through opening 14 of support 15 into absorbent 11. Absorbent 11 becomes in a humid condition with this dissolution liquid 16 and drug 10 is thoroughly activated. This pressure applied on dissolution liquid reservoir 18 pushes the whole device toward the skin side at the same time, and microneedle 51 perforates the skin (stratum corneum). The drug activated by this goes through solution passage 52 of microneedle substrate 53 and microneedle 51 and permeates into the skin. Dissolution liquid reservoir 18 becomes empty after dissolution liquid 16 has flowed out, and restores approximately the original shape as shown in Figure 2 (d).

Microneedle substrate 53 is constructed so that it has a strength not damaged when dissolution liquid reservoir 18 is pressed. The thickness of microneedle substrate 53 is about 0.1 to 3 mm, more preferably 0.5 to 2 mm when the material is silicon or metal material, and about 0.1 to 3 mm, more preferably 0.5 to 2 mm in the case of polymer material and the like as a substrate of laminate structure with reinforcing member. In this way, according to the present invention, movement of dissolution liquid is achieved at the same time with skin pricking and thus the applied pressure can be transmitted as skin pricking force as it is. Microneedle pricking force can be adjusted by changing the breaking force of diaphragm 20 by protruding part 17 of dissolution liquid reservoir 18. Specifically, the force when pressing to break the dissolution liquid reservoir is suitably, for example, in a range of 300 g to 3 kg/patch, preferably 500 to 2 kg/patch, and more preferably in a range of 700 to 1.5 kg/patch is proper. This is a value when it is assumed that the planar dimension of the needle preparation (microneedle substrate) is around 1 to 4 cm², and that the dissolution liquid reservoir is pressed for five seconds. In this way, according to the present invention, pressure applied on the dissolution liquid reservoir breaks the diaphragm provided between the dissolution liquid reservoir and the absorbent, and at the same time makes the microneedle to perforate the skin (stratum corneum), and thereby transmitting the drug dissolved in dissolution liquid through a microneedle device to the skin efficiently.

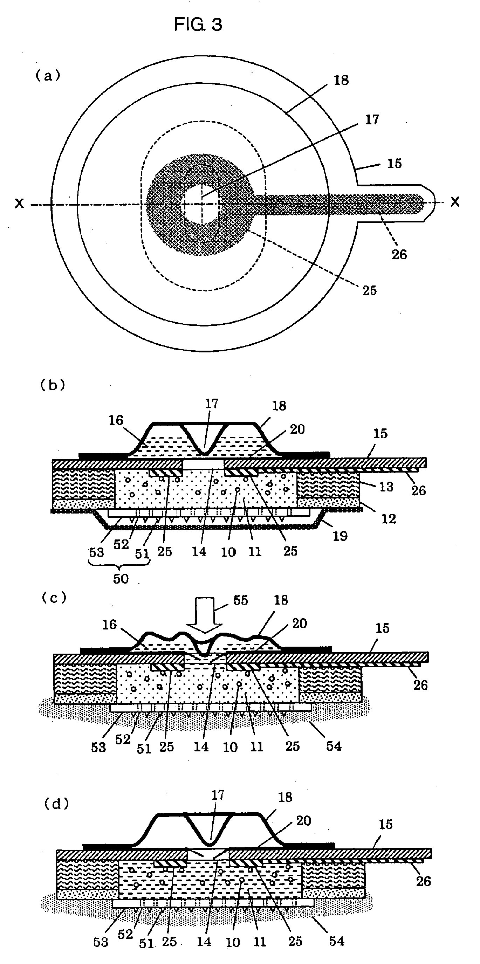

Figure 3 is a drawing showing another example of a transdermal drug administration system with microneedles of the present invention. (a) is a plan view, (b) is a cross-section view along X-X and (c) and (d) are drawings of the device of the present invention at use. Symbols in Figure 3 which are the same as in Figure 2 refer to the same object as in Figure 2. The point where this example is different from the example of Figure 2 is that electrode 25 to supply electric energy from an external part is possessed on absorbent 11. Lead part 26 is connected to electrode 25. This enables the device to be used as a device for electric drug administration system, for example, a device for iontophoresis system (iontophoresis electrode structural body). Except that, this is similar to the example of Figure 2.

Electrode 25 and lead part 26 can be made, for example, by printing them on the bottom surface of support 15. Electrode 25 is connected through lead part 26 to one output terminal (for example, + terminal) of the power-supply unit not shown. The other output terminal (for example, - terminal) of power-supply unit is connected to the counter device not shown. The counter device can be constructed similarly to the present transdermal drug administration system, but the counter device does not necessarily have to contain a drug. Electric voltage for iontophoresis or an electrical current is given between the present transdermal drug administration system and the counter device from a power-supply unit.

In time of use, liner 19 is removed and the present device (iontophoresis electrode structure body) is adhered onto skin 54. First, the top surface of dissolution liquid reservoir 18 is pressed in the direction of arrow 55 to break diaphragm 20 with protruding part 17 as shown in Figure 3 (c). At this time, diaphragm 20 is largely broken along the linear tip of protruding part 17 and the dissolution liquid in dissolution liquid reservoir 18 flows through opening 14 of support 15 into absorbent 11. Absorbent 11 becomes in a humid condition with this dissolution liquid and drug 10 is thoroughly activated. This pressure applied on dissolution liquid reservoir 18 pushes the whole device toward the skin side at the same time, and microneedle 51 perforates the skin (stratum corneum). And the power-supply unit not shown is turned on to start iontophoresis system. The drug activated by this goes through solution passage 52 of microneedle substrate 53 and microneedle 51 and permeates into the skin. Dissolution liquid reservoir 18 becomes empty after dissolution liquid 16 has flowed out, and restores approximately the original shape as shown in Figure 3 (d). In this way, according to the present invention, pressure applied on the dissolution liquid reservoir breaks the diaphragm provided between the dissolution liquid reservoir and the absorbent, and at the same time makes the microneedle to perforate the skin (stratum corneum), and thereby transmitting the drug dissolved in dissolution liquid through a microneedle device to the skin efficiently.

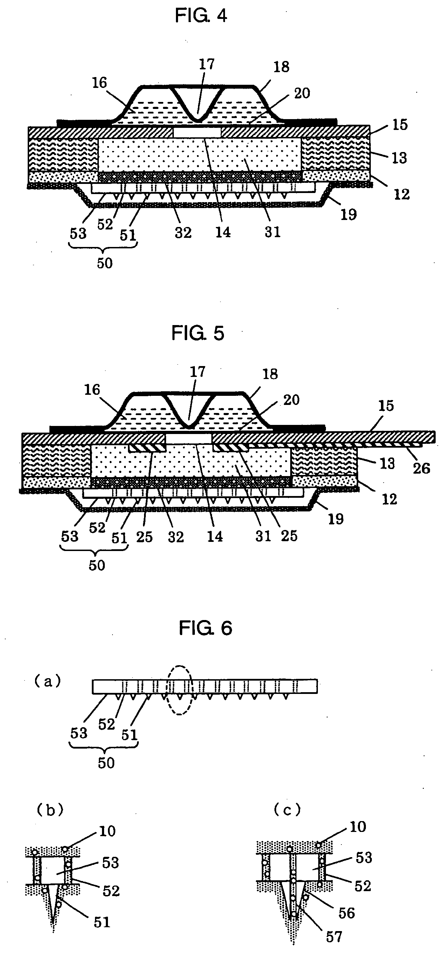

Figure 4 is a drawing showing another example of a transdermal drug administration system with microneedles of the present invention. The device of this example divides absorbent 11 containing a drug of Figure 2 into two, i.e., absorbent 31 which does not contain a drug and drug retaining member 32 which contains a drug, and the other is similar to the example of Figure 2. The reason why divided into absorbent 31 and drug retaining member 32 is to let the drug contact with the living body at a high concentration to make the drug absorption to the maximum.

Figure 5 is a drawing showing another example of a transdermal drug administration system with microneedles of the present invention. Symbols in Figure 5 which are the same as in Figures 3 to 4 refer to the same object as in Figures 3 to 4. The point where this example is different from the example of Figure 4 is that electrode 25 to supply electric energy from an external part is possessed on absorbent 11. Lead part 26 is connected to electrode 25. This enables the device to be used as a device for electric drug administration system, for example, a device for iontophoresis system (iontophoresis electrode structural body). Except that, this is similar to the example of Figures 3 to 4.

Figure 6 is a drawing showing a construction example of a microneedle device to be used in the transdermal drug administration system with microneedles of the present invention, and (a) is a whole view, (b) is a enlarged view of a part surrounded with a dotted line in (a), and (c) shows a partially enlarged view showing a modified example of a microneedle device. Microneedle device 50 comprises a plurality of microneedle 51 which can perforate the skin and microneedle substrate 53 having a plurality of solution passages 52 as shown in Figure 6(a). The dissolved drug 10 flows with the dissolution liquid through solution passage 52 to the skin along microneedle 51 as shown in Figure 6(b). In addition, hollow passage 57 which can transmit a drug in the direction along the length of microneedle 56 may be formed as shown in Figure 6(c) to connect solution passage 52 of microneedle substrate and hollow passage 57 of microneedle.

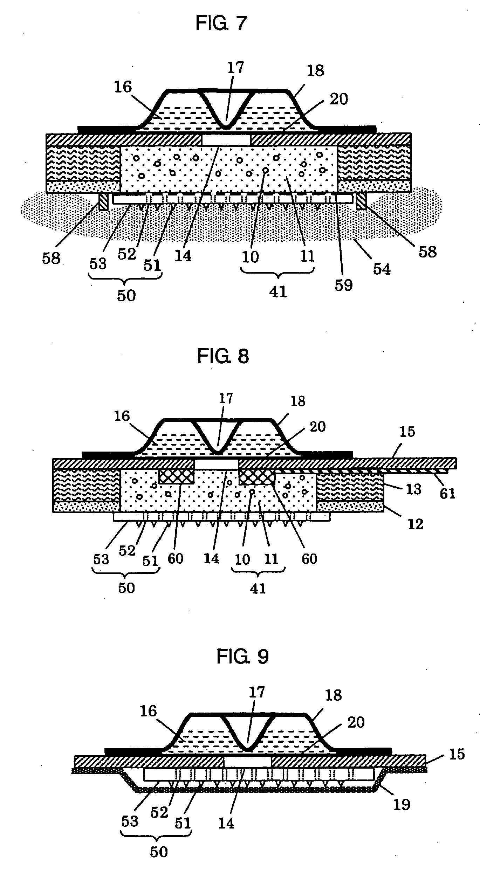

Figure 7 is a drawing showing another example of a transdermal drug administration system with microneedles of the present invention. The device of this example is provided with a skin fixation part 58 to extend skin at the microneedle pricking part outside microneedle device 50 of Figure 1 and further provided with a plate-like reinforcing part 59 having at least one solution passage in microneedle device 50, but the other is similar to the example of Figure 1. It is preferable that the height of skin fixation part 58 is larger than the thickness of microneedle device 50. In addition, skin fixation part 58 can be disposed outside adhesive layer 12 of microneedle device 50, but it is not limited to this. The shape can be made in the form of a ring, for example, an O-ring, but it is not limited to this and a part of a ring can be used and a form other than ring can be used. Plate-like reinforcing member 59 of microneedle device 50 is disposed, for example, on microneedle substrate 53. This is provided for reinforcing lest microneedle substrate 53 should be damaged. Because the skin is extended by skin fixation part 58, according to this example, it is easy for microneedle 51 to perforate the skin and thus this is advantageous in that microneedle device 50 can be also robust by plate-like reinforcing member 59. In this example, an example in which both skin fixation part 58 and plate-like reinforcing member 59 are provided in a device of Figure 1 is shown, but only one of these may be provided. In addition, skin fixation part 58 and/or plate-like reinforcing member 59 can be similarly provided in the devices of Figure 2 to Figure 5.

Figure 8 is a drawing showing another example of a transdermal drug administration system with microneedles of the present invention. The device of this example possessed sonic oscillator 60 on pad part 41 of Figure 1 to supply sound vibration energy from an external part and lead region 61 for connecting outside electric source. Sonic oscillator 60 is a doughnut form and is disposed, for example, surrounding opening 14 of support 15. Sonic oscillator 60 consists of a material such as ceramics, for example, and the vibration frequency is 1 KHz to 5 MHz and the intensity is up to 3.0 mW/cm². Sonic oscillator 60 is effective topromote diffusion of drug 10.

Figure 9 is a drawing showing another example of a transdermal drug administration system with microneedles of the present invention. The device of this example comprises microneedle device 50 comprising microneedle substrate 53 having a plurality of microneedles 51 which can perforate the skin and dissolution liquid reservoir 18 disposed on microneedle device 50 and storing dissolution liquid 16 for dissolving a drug. In this example, at least one solution passage 52 is formed in microneedle substrate 53. Dried drug is disposed in microneedle device 50. Specifically, the dried drug is disposed, for example, at least one of top surface, bottom surface of needle substrate 53 and solution passage 52. When the dried drug is disposed on the bottom surface of microneedle substrate 53, it may be disposed in microneedle 51. In time of use, liner 19 is removed and the device is put on the skin, and diaphragm 20 is destroyed by pressing protruding part 17 of dissolution liquid reservoir 18, and dissolution liquid reservoir 18 is opened and dissolution liquid 16 flows through opening 14 formed in support 15 and supplied to microneedle device 50. This allows dissolution liquid 16 to go through solution passage 52 formed in microneedle substrate 53 and supplied to microneedle 51. At the same time, microneedle 51 perforates a stratum corneum of the skin and thereby the drug dissolved in the dissolution liquid is transdermally absorbed. In this drawing, an adhesive layer to keep liner 19 in support 15 before use and drug disposed on microneedle device 50 are omitted for simplification.

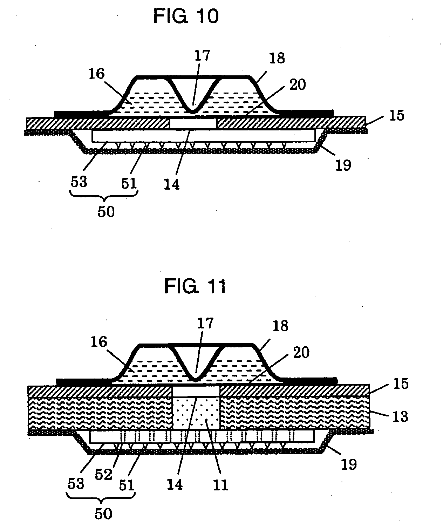

Figure 10 is a drawing showing another example of a transdermal drug administration system with microneedles of the present invention. The device of this example is different from the example of Figure 9 at the point where no solution passage is formed in microneedle substrate 53, but the other is similar to the example of Figure 9. That is, in this example, liner 19 is removed in time of use and the device is put on the skin, and diaphragm 20 is destroyed by pressing protruding part 17 of dissolution liquid reservoir 18, and dissolution liquid reservoir 18 is opened and dissolution liquid 16 flows through opening 14 formed in support 15 and supplied to microneedle device 50. At this time, dissolution liquid 16 permeates into microneedle substrate 53 oppositely facing opening 14, and dissolution liquid 16 is supplied to microneedle 51 from the circumference of microneedle substrate 53. At the same time, microneedle 51 perforates a stratum corneum of the skin and thereby the drug dissolved in the dissolution liquid is transdermally absorbed. Because microneedle substrate 53 of this example does not form solution passage 52 such as in the example of Figure 9, this is advantageous in that constitution is simple and manufacturing is easy. A ditch for flowing the dissolution liquid, however, may be formed on at least one of the top and bottom surfaces of needle substrate 53 so as to make the dissolution liquid 16 easy to flow into microneedle 51 from the circumference of microneedle substrate 53. In addition, a certain clearance may be formed between dissolution liquid reservoir 18 and microneedle substrate 53 without closely contacting them so that dissolution liquid 16 is easy to permeate.

Figure 11 is a drawing showing another example of a transdermal drug administration system with microneedles of the present invention. The device of this example is different from the example of Figure 9 in that absorbent 11 which consists of a material capable of absorbing liquid is surrounded with wall members 13 at least on the part where dissolution liquid reservoir 18 is opened between microneedle device 50 and dissolution liquid reservoir 18, but the other is similar to the example of Figure 9. That is, in this example, liner 19 is removed in time of use and the device is put on the skin, and diaphragm 20 is destroyed by pressing protruding part 17 of dissolution liquid reservoir 18, and dissolution liquid reservoir 18 is opened and dissolution liquid 16 flows through opening 14 formed in support 15 and through absorbent 11 provided on the corresponding part and supplied to microneedle device 50. Due to this, dissolution liquid 16 is supplied to microneedle 51 through solution passage 52 formed in microneedle substrate 53. At the same time, microneedle 51 perforates a stratum corneum of the skin and thereby the drug dissolved in the dissolution liquid is transdermally absorbed. In this example, construction in which solution passage 52 is formed is used as microneedle substrate 53 in the same way as in the example of Figure 9 but not limited to this and, for example, construction in which no solution passage is formed in microneedle substrate 53 can be used in the same way as in the example of Figure 10. In this case, dissolution liquid 16 is supplied to microneedle 51 from the circumference of microneedle substrate 53 as mentioned above.

In the examples shown in Figures 9 to 11, although not shown in the drawings, an electrode to supply electric energy from an external part can be provided in the microneedle device or the absorbent. This enables the device to be used as a device for electric drug administration system, for example, a device for iontophoresis system (iontophoresis electrode structural body). In addition, a sonic oscillator can be provided on the microneedle device or the absorbent in order to supply sound vibration energy from an external part. In addition, a plurality of microneedles may have hollow passages 57 which can transmit a drug in the direction along the length of microneedles to connect solution passage of microneedle and hollow passage of microneedle substrate. Furthermore, the microneedle device can possess a skin fixation part on the outside to extend the skin.

The following materials can be used in each part of a transdermal drug administration system with microneedles of the present invention.

As for the physiologically active substance (drug), various kinds of drugs which accord to the purpose of treatment can be selected, and, for example, types of the drug, types of salts, application of each drug and the like are not limited as long as it is a compound having pharmacological activity, and, for example, antibiotic drug, antifungal drug, antitumor agent, cardiotonic drug, antiarrhythmic, vasodilator, antihypertensive drug, diuretic, depression diuretic, circulation ingeniousness agent, antiplatelet, hemostatic drug, hypolipidemic drug, alleviation of fever/painkilling/antiphlogistic agent, antirheumatic, relaxant, antitussive and expectorant, antiulcer agent, sedative, antiepileptic drug, antidepressant, antiallergic drug, diabetes therapeutic agent, tuberculostatic agent, hormone drug, narcotic antagonist, bone resorption depressant, vascularization inhibitor, local anesthetic, etc. are used.

In the case of a device to be used in iontophoresis system, various kinds of drugs which accord to the purpose of treatment can be selected, but, on the occasion of the medication using iontophoresis, it is particularly useful for drugs for which permission of precision of medication quantity is severe. For example, the present device can be safely used for drugs having a narrow width between the effective blood concentration and side effect exhibiting density such as insulin. In addition, suppressing electric error factors as much as possible is important to obtain high safety and effectiveness of drug even for the other drugs having a wide width between the effective blood concentration and side effect exhibiting density

In addition to drugs, dissolution rate modifier of drugs, additive for stabilization, adsorption inhibitor, etc. can be added. PH regulator, penetration enhancer are held with dry state appropriately.

For the absorbent, materials which can absorb liquid well are selected, and examples thereof include polyester (polyethylene terephthalate), polysaccharides or cellulosic derivative (rayon, cotton), polyamide (nylon), non-woven cloth, woven cloth, gauze or porous body such as sponges or hydrophilic polymer (agar, agarose, algic acid, xanthan gum, guar gum, dextran, dextrin, pullulan, chitosan, gelatine, carboxyvinyl polymer, polyacrylate, carboxymethylcellulose salt, polyoxyalkylene, polyvinyl alcohol, polyvinylpyrrolidone, polyacrylamide), ion-exchange resin (amberlite, diaion, cholestyramine), and preferred, for example, is a nonwoven mainly composed of rayon.

As for the drug retaining member, for example, hydrophilic film, or rigid materials such as ceramics, metals and polymer materials in which a passage capable of flowing a drug is formed can be used. In addition, those in which a porous film or ion exchange membrane contains a drug can be also used. Examples of the porous film include PE, PP, cellulose, cellulose acetate, PET, nylon and the like. Examples of ion-exchanged film, cation-exchanger membrane, anion-exchange membrane, complex charged film are given, but preferred is nylon cation exchange membrane.

As for the wall member, materials of non-water permeability are selected, and examples thereof include foaming polyolefin (PE, PP) foaming polyurethane, styrofoam, foam rubber (polybutyren), foaming EVA, foaming PVC, and preferably, for example, it is foaming polyolefin.

Example of the adhesive layer include natural rubber, styrene-isoprene-styrene block copolymer, styrene-butadiene rubber, styrene-isoprene rubber, polyisobutyrene, polyisoprene, polyacrylates, silicone rubber, and preferably, for example, acrylates.

As for the support, non-water permeable materials are selected, and, examples thereof include polyolefin, polyurethane, polystyrene, rubber, EVA, PVC, PET are given.

Examples of the dissolution liquid reservoir include molded sheet materials composed of a laminate of PET, PVC, PVDC, PP, PE, polystyrene, cyclic polyolefin (COC), Al and these in the shape of a dome and a convex protruding part is formed therein, or sheets having highly barrier properties (PCTFE/PP, PCTFE/PVC, cyclic polyolefin/PP), Al deposited or SiO₂ deposited sheets. By pressing a convex protruding part of the dissolution liquid reservoir, diaphragm or a laminate of diaphragm and support is destroyed at least one point. As for the convex protruding part, destroyed part becomes a point with form of cone, and penetration of the dissolution liquid to the absorbent side becomes bad. A convex breakable part (tip of protruding part) is preferably linear or a form of surface. Materials thereof may be PCTFE (-CF₂-CFCl-)ₙ poly (chloro-trifluoroethylene), COC cyclic polyolefin copolymer. The thickness of a sheet is, for example, 100 to 500µm. PP, PP/COC/PP, PCTFE/PParepreferably, for example, used for a dissolution liquid reservoir.

Examples for the diaphragm (membrane to be broken with a protruding part) include Al, PP, PE and laminates of these. It is preferable to perform coating to prevent corrosion if necessary for Al foil. The thickness of diaphragm is, for example, 5 to 100 µm for Al and 15 to 50 µm for PP and PE.

Examples of the dissolution liquid include water, alcohol, polyalcohol, surfactant, saccharides, pH regulator (organic and inorganic acid/base), salts, water-soluble polymer, solvent, penetration enhancer, oils and fats, a preservative, but preferably, for example, purified water, glycerin, methylparaben, (propylparaben, propylene glycol).

Examples of the liner include PET, PEN, PP, PE, paper, Al, laminates of these, but preferably, it is PET. In addition, it is preferable to perform releasing treatment such as siliconization. Furthermore, it is preferable to process liner concavely not to come in contact with microneedle.

In addition, according to the present invention, solution permeable film can be provided on the bottom surface of an absorbent containing a drug in the examples of Figure 2 and Figure 3. The solution permeable film is effective to maintain an absorbent and functions as retaining means for the case containing a powdered material. For example, for the solution permeable film, porous film or ion exchange membrane can be used. Examples of the porous film include PE, PP, cellulose, cellulose acetate, PET, nylon. Examples of the ion-exchanging membrane include cation-exchanging membrane, anion-exchange membrane, complex charged film, but preferably it is cation-exchanging membrane of nylon. However, when the absorbent is a nonwoven, the solution permeable film is not necessary.

### Industrial Applicability

The present invention relates a transdermal drug administration system for administering a drug through the skin, and particularly relates to a transdermal drug administration system with microneedles comprising a plurality of microneedles which can perforate the skin, and it has industrial applicability.

## Claims

1. A transdermal drug administration system with microneedles comprising: a microneedle device having a plurality of microneedles which can perforate the skin and a microneedle substrate having at least one solution passage; a pad part disposed on the microneedle device; andadissolution liquid reservoir disposed on the pad part and storing dissolution liquid for dissolving a drug, wherein a dried drug is placed in the pad part or microneedle device, and the dissolution liquid reservoir is pressed to open the dissolution liquid reservoir from a sealed state to supply the dissolution liquid to the pad part and to allow the microneedles to perforate the stratum corneum of the skin, thereby enabling the drug dissolved in the dissolution liquid to be transdermally absorbed.

2. The transdermal drug administration system with microneedles according to claim 1, wherein an electrode to supply electric energy from an external part is provided on the pad part.

3. The transdermal drug administration system with microneedles according to claim 1 or 2, wherein a sonic oscillator to supply sound vibration energy from an external part is provided on the pad part.

4. The transdermal drug administration system with microneedles according to any of claims 1 to 3, wherein the microneedle device comprises a plate-like reinforcing member having at least one solution passage on the microneedle substrate.

5. The transdermal drug administration system with microneedles according to any of claims 1 to 4, wherein the pad part comprises a drug retaining member which contains the dried drug and an absorbent which absorbs the dissolution liquid.

6. A transdermal drug administration system with microneedles comprising: a microneedle device comprising a plurality of microneedles which can perforate the skin and a microneedle substrate having at least one solution passage; an absorbent which is placed on the microneedle device, can contain the above dried drug and comprises a material which can absorb the liquid; and a dissolution liquid reservoir which is disposed on the absorbent and stores dissolution liquid for dissolving the drug and in which a diaphragm provided between the reservoir and the absorbent can be broken by pressing.

7. A transdermal drug administration system with microneedles comprising: a microneedle device comprising a plurality of microneedles which can perforate the skin and a microneedle substrate having at least one solution passage; a drug retaining member which is disposed on the microneedle device and contains a dried drug; an absorbent which is placed on the drug retaining member and comprises a material which can absorb the liquid; and a dissolution liquid reservoir which is disposed on the absorbent and stores dissolution liquid for dis solving the drug and in which a diaphragm provided between the reservoir and the absorbent can be broken by pressing.

8. The transdermal drug administration system with microneedles according to claim 6 or 7, wherein an electrode to supply electric energy from an external part is provided on the absorbent.

9. The transdermal drug administration system with microneedles according to claim 6 or 7, wherein a sonic oscillator to supply sound vibration energy from an external part is provided on the absorbent.

10. The transdermal drug administration system with microneedles according to claim 9, wherein a plurality of the microneedles have a hollow passage which can transmit the drug in the direction along the length, and the hollow passage of the microneedle is connected with a solution passage of the microneedle substrate.

11. The transdermal drug administration system with microneedles according to any of claims 6 to 10, wherein the microneedle comprises a skin fixation part on the outside the device to extend the skin.

12. A transdermal drug administration system with microneedles comprising: a microneedle device comprising a plurality of microneedles which can perforate the skin and a microneedle substrate having at least one solution passage; a dissolution liquid reservoir which is disposed on the microneedle substrate and stores dissolution liquid for dissolving the drug, wherein a dried drug is placed in the microneedle device, and the dissolution liquid reservoir is pressed to open the dissolution liquid reservoir from a sealed state to supply the dissolution liquid to the microneedle device and to allow the microneedles to perforate the stratum corneum of the skin, thereby enabling the drug dissolved in the dissolution liquid to be transdermally absorbed.

13. The transdermal drug administration system with microneedles according to claim 12, wherein the dissolution liquid can be supplied to the microneedle through at least one solution passage formed on the microneedle substrate.

14. The transdermal drug administration system with microneedles according to claim 12, wherein the dissolution liquid can be supplied to the microneedle from the circumference of the microneedle substrate.

15. The transdermal drug administration system with microneedles according to any of claims 12 to 14, wherein an absorbent composed of a material which can absorb liquid is provided at least on the part where the dissolution liquid reservoir is opened between the microneedle device and the dissolution liquid reservoir.

16. A transdermal drug administration method comprising: applying a device which comprises a microneedle device having a plurality of microneedles which can perforate the skin; a pad part disposed on the microneedle device; a dissolution liquid reservoir disposed on the pad part and storing dissolution liquid for dissolving a drug; and drug disposed in the pad part or the microneedle device to the skin, and pressing the dissolution liquid reservoir to open the dissolution liquid reservoir from a sealed state to supply the dissolution liquid to the pad part and to allow the microneedles to perforate the stratum corneum of the skin, thereby transdermally administering the drug dissolved in the dissolution liquid through the microneedles.

17. A transdermal drug administration method comprising: applying a device which comprises a microneedle device having a plurality of microneedles which can perforate the skin; a dissolution liquid reservoir disposed on the microneedle device and storing dissolution liquid for dissolving a drug; and a drug disposed in the microneedle device to the skin, and pressing the dissolution liquid reservoir to open the dissolution liquid reservoir from a sealed state to supply the dissolution liquid to the microneedles and to allow the microneedles to perforate the stratum corneum of the skin, thereby transdermally administering the drug dissolved in the dissolution liquid through the microneedles.
